# EUROPÄISCHE PATENTSCHRIFT

(11) **EP 1 446 139 B1**
(45) Veröffentlichungstag und Bekanntmachung des Hinweises auf die Patenterteilung: **24.09.2008**
(21) Anmeldenummer: 02803402.3
(22) Anmeldetag: 21.11.2002
(51) Int. Cl.: A61K 38/17, A61P 37/00

(54) **PHARMAZEUTISCHE ZUSAMMENSETZUNG ENTHALTEND LÖSLICHEN FCGAMMA REZEPTOR IIB**
PHARMACEUTICAL COMPOSITION CONTAINING SOLUBLE FCGAMMA RECEPTOR IIB
COMPOSITION PHARMACEUTIQUE CONTENANT LE RECEPTEUR IIB SOLUBLE DE FCGAMMA

(30) Priorität: 22.11.2001 DE 10157290
(43) Veröffentlichungstag der Anmeldung: 18.08.2004
(73) Patentinhaber: MAX-PLANCK-GESELLSCHAFT ZUR FÖRDERUNG DER WISSENSCHAFTEN E.V., 80539 München (DE)
(72) Erfinder: HUBER, Robert, 82110 Germering (DE); SONDERMANN, Peter, 82152 Krailling (DE); JACOB, Uwe, 80339 München (DE)
(74) Vertreter: Böhm, Brigitte
(86) Internationale Anmeldenummer: PCT/EP2002/013080
(87) Internationale Veröffentlichungsnummer: WO 2003/043648

(56) Entgegenhaltungen:
- WO-A-00/32767
- WO-A-99/40117
- IERINO F L ET AL: "Recombinant soluble human Fc-gamma-RII: Production, characterization, and inhibition of the Arthus reaction." JOURNAL OF EXPERIMENTAL MEDICINE, Bd. 178, Nr. 5, 1993, Seiten 1617-1628, XP009015491 ISSN: 0022-1007

## Beschreibung

Die vorliegende Erfindung betrifft pharmazeutische Zusammensetzungen, welche den Rezeptor FcγR IIb in rekombinant hergestellter löslicher Form enthalten.

Fc-Rezeptoren (FcRs) spielen eine wichtige Rolle bei Abwehrreaktionen des Immunsystems. Nachdem Pathogene Zugang zum Blutkreislauf gefunden haben, werden sie von Immunglobulinen, den Antikörpern, gebunden. Die daraus entstehenden Immunkomplexe binden aufgrund ihrer Multivalenz mit den Fc-Fragmenten der Antikörper mit hoher Avidität an Fc-Rezeptor präsentierende Phagozyten, welche die Pathogene zerstören und eliminieren. Auch akzessorische Zellen wie natürliche Killerzellen, Eosinophile und Mastzellen tragen auf ihrer Oberfläche Fc-Rezeptoren, die nach der Bindung von Immunkomplexen gespeicherte Mediatoren, wie Wachstumsfaktoren oder Toxine, freisetzen, die die Immunantwort unterstützen.

Die Fc-Rezeptoren der akzessorischen Zellen sind daher Signalmoleküle und binden bei der humoralen Immunantwort spezifisch Immunglobuline verschiedener Isotypen. Des Weiteren können Fc-Rezeptoren natürliche Killerzellen dazu aktivieren, Antikörper-behaftete Zielzellen zu zerstören ("Antikörper-abhängige zellvermittelte Zytotoxizität", ADCC).

Neben den Pathogen-abwehrenden positiven Effekten von FcRs können allerdings auch überschießende Reaktionen auftreten, die bei gesunden Personen eine unerwünschte Stimulation des Immunsystems bewirken, welche sich insbesondere in Allergien oder Autoimmunerkrankungen äußern. Derartige, gegen körpereigene Substanzen gerichtete Immunreaktionen sind nach wie vor ein großes medizinisches Problem, für das es zwar Behandlungsansätze gibt, welche jedoch nicht bei jedem Patienten gleich gut wirken.

Auch mit dem Vorliegen erhöhter Konzentrationen von natürlichen Killerzellen (NK-Zellen), welche ebenfalls aufgrund überschießender Immunreaktionen gebildet werden, gehen medizinische Probleme einher. So ist beobachtet worden, dass bei schwangeren Frauen mit erhöhtem NK-Zellspiegel häufig Fehlgeburten vorkommen, da sowohl die Einnistung als auch das intrauterine Wachstum des Fötus behindert sind. Zur Behandlung von schwangeren Frauen mit erhöhten NK-Zellspiegeln werden bisher hochdosierte Immunglobuline auf intravenösem Weg verabreicht. Durch dieses intravenös verabreichte Immunglobulin G (IVIg) sollen NK-Zellen neutralisiert und die überschießende Immunantwort gedämpft werden. Auch bei Autoimmunerkrankungen (z.B. Multiple Sklerose) wurden IVIg bereits verabreicht. Allerdings verursacht die Verabreichung von IVIg in den notwendigen hohen Dosierungen erhebliche Probleme. Um eine ausreichende IVIg-Konzentration im Serum zu erreichen, müssen große Mengen dieses Proteins infundiert werden (25 - 150 g), was über ein relativ großes Volumen geschieht (250 - 1500 ml). Diese große Flüssigkeitsmenge' muss über 2 bis 4 Stunden infundiert werden, eine schnellerre Verabreichung verstärkt die dabei ohnehin beobachteten Nebenwirkungen, wie Kopfschmerzen, Fieber, allgemeines Missempfinden und dgl. Die Verabreichung ist an 1 bis 3 aufeinanderfolgenden Tagen nötig, diese Behandlung ist jeweils einmal im Monat durchzuführen.

Neben dem erheblichen Zeitaufwand und den Nebenwirkungen einer solchen IVIg-Behandlung besteht der weitere Nachteil, dass die Behandlung sehr teuer ist, die während einer einzigen Schwangerschaft ca. US$ 10.000 kostet. Die Behandlungskosten müssen außerdem in der Regel von der Patientin selbst getragen werden. Die gleichen Nachteile hat eine IVIg-Behandlung mit entsprechender Dosierung bei z.B. Multipler Sklerose oder anderen neurologischen Erkrankungen (Achiron et al., Neurology (1998), 50(2): 398-402; Dalakas, Ann Intern Med. (1977), 126(9): 721-30; Brannagan et al., Virology (1996), 47(3): 674-7). Die WO 00/32767 beschreibt lösliche Fc-Rezeptoren, welche lediglich aus dem extrazellulären Teil des Rezeptors bestehen und nicht glykosyliert sind. Aufgrund des Fehlens der Transmembrandomäne und des Signalpeptids liegen diese Proteine in löslicher Form und nicht zellgebunden vor. Die dort beschriebenen Fc-Rezeptoren können außerdem rekombinant hergestellt werden und sind zur Behandlung von Autoimmunerkrankungen vorgeschlagen worden, da sie Antikörper binden können, jedoch keine Effektorwirkung auf andere Komponenten des Immunsystems ausüben. Sie haben dadurch die Fähigkeit, Antikörper im Blutstrom zu neutralisieren, was sich insbesondere auf Autoimmunprozesse dämpfend auswirkt. Die WO 00/32767 beschreibt außerdem die Kristallstruktur bestimmter Fc-Rezeptoren sowie die Möglichkeit der Auffindung von Substanzen, die die Interaktion von IgG mit Fc-Rezeptoren inhibieren, mithilfe von Kristallstrukturen. Die Aufklärung der Kristallstruktur erlaubt es, über das Screening von vorhandenen Datenbanken mithilfe von Computerprogrammen entsprechende Inhibitoren aufzufinden.

Der vorliegenden Erfindung lag die Aufgabe zugrunde, die Erkenntnisse der WO 00/32767 weiterzuentwickeln und insbesondere für die Indikationen Multiple Sklerose (MS), Systemischer Lupus erythematosus (SLE) und Rheumatoide Arthritis (RA), jedoch auch bei Erkrankungen mit erhöhtem NK-Zellspiegel, Behandlungsmöglichkeiten bereitzustellen, welche Nachteile der bisherigen Behandlungsverfahren vermeiden, leicht anzuwenden und preisgünstig durchzuführen sind.

Diese Aufgabe wurde im Rahmen der vorliegenden Erfindung gelöst durch pharmazeutische Zusammensetzungen in Form von wässrigen Lösungen, welche rekombinant hergestellten, löslichen FcγR IIb in einer Menge von 40 bis 4000 mg, bevorzugt 100 bis 1000 mg und einer Konzentration von bis zu 50 mg/ml (vorzugsweise z.B. 8 ml pro Injektion) enthalten. Im Rahmen der vorliegenden Erfindung wurde festgestellt, dass die beiden genannten Rezeptoren, welche rekombinant in Prokaryonten hergestellt wurden und daher unglykosyliert sind, also schlechter löslich sein sollten, überraschenderweise dennoch auch mit bekannten Methoden so aufgereinigt werden können, dass eine relativ hohe Konzentration von sFcR in gelöster Form erhalten werden kann. Weiterhin wurde festgestellt, dass diese Rezeptoren bereits in einer relativ geringen Dosierung und insbesondere in der Dosierung von 0,5 mg/kg bis 50 mg/kg Körpergewicht außerordentlich starke Effekte bei der Bekämpfung von überschießenden Immunreaktionen zeigen.

Durch die Erhältlichkeit in hochkonzentrierter löslicher Form wird es ermöglicht, die erfindungsgemäße pharmazeutische Zusammensetzung zu injizieren, langwierige Infusionen über die Dauer von mehreren Stunden, wie bei IVIg Behandlungen üblich, können bei Verwendung der erfindungsgemäßen pharmazeutischen Zusammensetzung vermieden werden. Die erfindungsgemäße pharmazeutische Zusammensetzung weist darüber hinaus im Allgemeinen Träger- oder/und Hilfsstoffe auf, welche pharmazeutisch akzeptierbar sind und pharmakologisch die Anwendung der pharmazeutischen Zusammensetzung unterstützen oder erleichtern.

Die pharmazeutische Zusammensetzung enthält bevorzugt den FcγR IIb Rezeptor, weicher mindestens die in SEQ ID NO. 1 angegebene Aminosäuresequenz umfasst. Diese Sequenz stellt eine Minimalsequenz dar, die prinzipiell mit geeigneten Sequenzen der Wildtyp-Proteine an den Termini verlängert werden kann Bevorzugt wird bei Verlängerung der N- oder/und C-Termini der angegebenen Sequenzen keine Mutation in die Konstrukte eingeführt, um einer Antigenizität vorzubeugen. Theoretisch können jedoch auch Mutationen oder Deletionen in die verlängerten Sequenzen eingeführt werden, solange dadurch nicht unerwünschte Antigenizität hervorgerufen wird.

Ein Beispiel für eine an den Termini verlängerte Sequenz mit Anteilen von Signal- oder/und Linkersequenzen, die zwar zum Gen gehören, aber für ein therapeutisches Protein nicht unbedingt notwendig sind, ist für FcγR IIb die SEQ ID NO. 3.

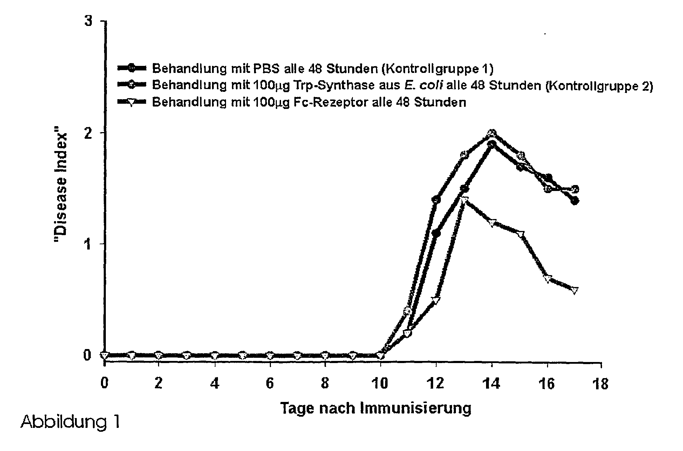

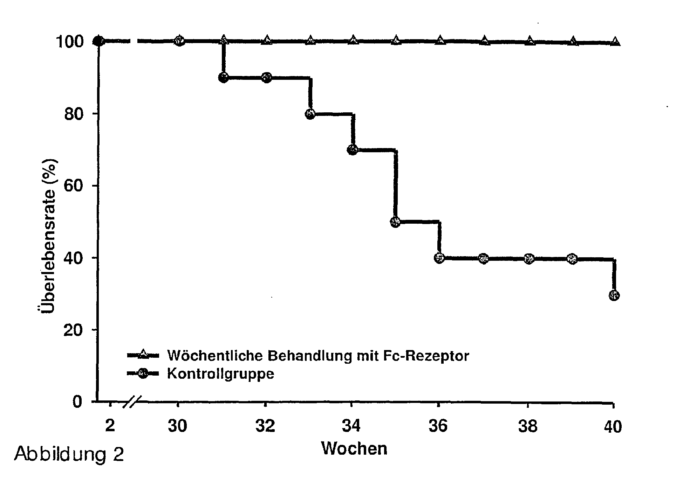

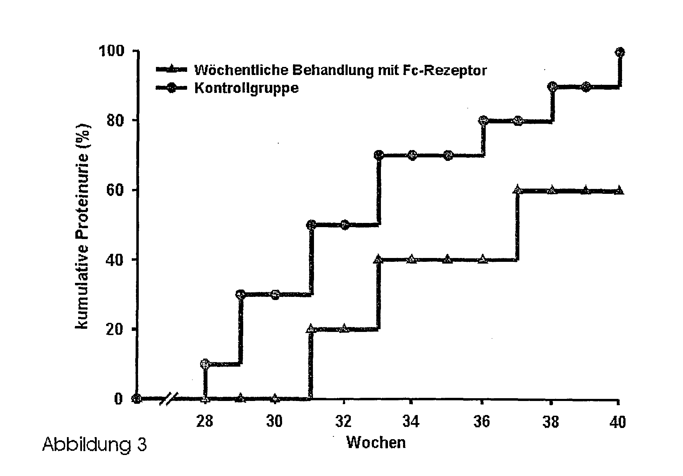

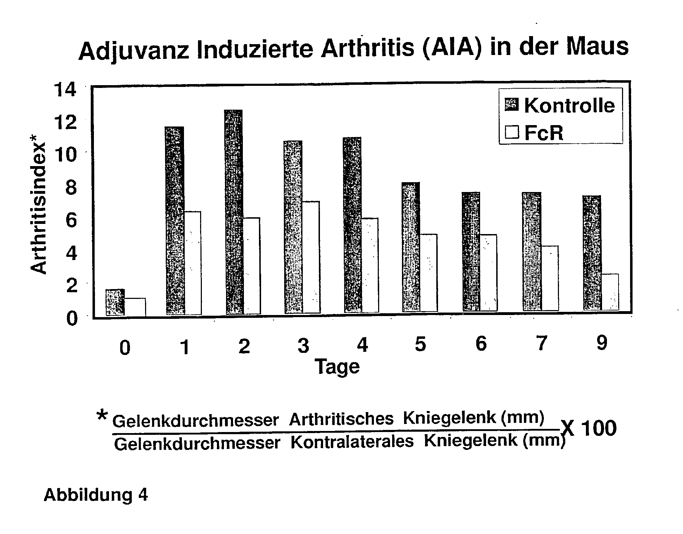

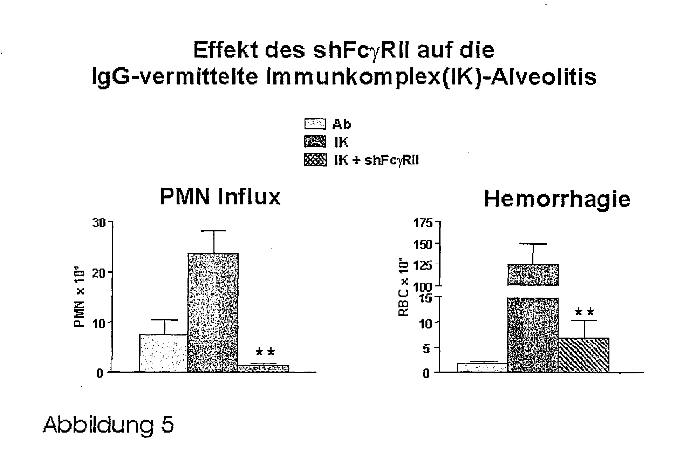

Der Rezeptor FcγR IIb mit der in SEQ ID NO.1 bzw. 3 gezeigten Aminosäuresequenz hat sich als außerordentlich wirksam bei der Anwendung bei SLE, MS und RA erwiesen. Abbildung 1 zeigt, dass bei MS im Vergleich zu Kontrollproben bei Verabreichung von FcγR IIb der "Disease Index" (ein Wert, der den Grad der Erkrankung wiedergibt und für den mehrere definierte klinische Parameter zur Einstufung benutzt werden) deutlich niedriger liegt als bei Kontrollen. Bei der Behandlung von Mäusen, welche Symptome einer Lupuserkrankung aufweisen, konnte ebenfalls bei Verabreichung von FcγR IIb gemäß vorliegender Erfindung die Überlebensrate deutlich gesteigert werden im Vergleich zu einer Kontrollgruppe (Abbildung 2). Auch der Anstieg der Proteinurie, welche als Maß für die Verschlechterung des Zustandes der Mäuse gewertet werden kann, konnte bei Verabreichung von FcγR IIb deutlich verlangsamt werden (Abbildung 3). Weitere Beispiele für die Anwendung des löslichen FcγR IIb sind die Rheumatoide Arthritis (Abbildung 4) sowie Krankheiten, die mit hoher Immunkomplexlast einhergehen, wie hier am Beispiel der Immunkomplex-induzierten Alveolitis gezeigt (Abbildung 5). Insgesamt kann FcγR IIb den Verlauf von Krankheiten mit Immunkomplexbeteiligung und insbesondere von Autoimmunerkrankungen, wie Multiple Sklerose, SLE oder Rheumatoider Arthritis, eindeutig positiv beeinflussen und dies bereits bei Verabreichung von äußerst geringen Mengen des löslichen Rezeptors. Es muss daher davon ausgegangen werden, dass ein neuer Wirkmechanismus aufgefunden wurde, der dadurch erklärt werden könnte, dass nicht alle Antikörper durch Fc-Rezeptorbindung eliminiert werden, sondern präferentiell die in Immunkomplexen gebundenen Autoantikörper oder aber durch einen neuen, bisher unbekannten regulatorischen Mechanismus in das Krankheitsgeschehen eingreift.

Die Kosten für die erfindungsgemäßen pharmazeutischen Zusammensetzungen liegen dabei außerdem sehr niedrig, da die Herstellung durch rekombinante Expression in Prokaryonten einfach ist und zu hochgereinigten und hochkonzentrierten Proteinpräparationen führt. Insofern können die pharmazeutischen Zusammensetzungen der vorliegenden Erfindung zu einem Bruchteil der Kosten hergestellt werden, welche für IVIg-Präparationen veranschlagt werden müssen.

Ein weiterer Gegenstand der vorliegenden Erfindung ist die Verwendung von erfindungsgemäßen pharmazeutischen Zusammensetzungen zur Behandlung von überschießenden Immunreaktionen, und insbesondere zur Behandlung von Multipler Sklerose, SLE, RA oder Patienten, welche eine erhöhte Zahl von natürlichen Killerzellen im Blutstrom aufweisen.

Es ist dabei, wie oben bereits beschrieben, besonders bevorzugt, das erfindungsgemäße Arzneimittel in injizierbarer Form anzuwenden, da es die erfindungsgemäße pharmazeutische Zusammensetzung mit ihrer speziellen Dosierung und Konzentration erlaubt, ausreichende Mengen von löslichen Fc-Rezeptoren auch auf diese Weise zu verabreichen. Es ist dabei wiederum besonders bevorzugt, den entsprechenden FcR der SEQ ID NO.1 oder durch Wildtyp-Sequenzen am N- oder/und C-Terminus verlängerte Proteine, insbesondere solche gemäß SEQ ID NO. 3, zu verwenden. Die FcRs selbst können gemäß der Beschreibung der WO 00/32767 in Prokaryonten exprimiert sowie danach gereinigt und zurückgefaltet werden. Beide Rezeptoren weisen den Vorteil auf, dass sie hochkonzentrierbar sind, sodass lediglich geringe Volumina nötig sind, um ausreichend wirksame Mengen zu verabreichen. Außerdem wurde im Rahmen der vorliegenden Erfindung, wie oben bereits ausgeführt, festgestellt, dass beide Rezeptoren offensichtlich nicht kompetitiv wirken, sondern ein neuer Wirkungsmechanismus abläuft, der es bedingt, dass bereits bei Verabreichung geringer Mengen der Rezeptoren positive Wirkungen erreicht werden. Die erfindungsgemäßen pharmazeutischen Zusammensetzungen und ihre Verwendung für die Behandlung von Erkrankungen, welche auf überschießenden Immunreaktionen basieren, sind daher aufgrund ihrer Dosierung und Verabreichbarkeit besonders vorteilhaft und für die fortgesetzte Behandlung auch aufgrund ihrer geringen Herstellungskosten besonders gut geeignet.

### Die folgenden Figuren erläutern die Erfindung:

### Abb. 1: Krankheitsverlauf von induzierter EAE in DBA/1 Mäusen

Die experimentelle allergische Enzephalomyelitis (EAE) in DBA/1 Mäusen stellt ein Tiermodell für Multiple Sklerose (MS) dar. Die MS-artigen Symptome in diesem Mausstamm (z.B. Lähmungserscheinungen, Läsionen im Gehirn) wird nach Immunisierung mit Myelin-Oligodendrozyten-Glykoprotein (MOG) beobachtet. Je 10 derart immunisierte DBA/1 Mäuse wurden in 48 stündigem Rhytmus mit PBS (Kontrolle 1), 100µg Trp-Synthase aus E.coli (Kontrolle 2) oder 100µg löslichem Fc-Rezeptor (sFcγRIIb) behandelt. Die Symptome der EAE wurden individuell bewertet und als Gruppendurchschnitt aufgetragen. Der "Disease Index" (Nach: Abdul-Majid, K. B., Jirholt, J., Stadelmann, C., Stefferl, A., Kjellen, P., Wallstrom, E., Holmdahl, R., Lassmann, H., Olsson, T., Harris, R. A. (2000), Screening of several H-2 congenic mouse strains identified H-2(q) mice as highly susceptible to MOG-induced EAE with minimal adjuvant requirement. J. Neuroimmunol. 111: 23-33) wird wie folgt bewertet: 0, keine Zeichen von EAE, 1, Schwanz-Paralyse; 2, Erschlaffung der Hinterbeine; 3, Hinterbeinlähmung; 4, Komplette Lähmung der Vorder- und Hinterbeine; 5, sterbend.

### Abb. 2: Überlebensrate von NZBW/F₁ Mäusen

NZBW/F₁ Mäuse stellen ein anerkanntes Tiermodell für die Krankheit Systemischer Lupus Erythematosus (SLE) dar (Theofilopoulos, A. N., Dixon, F. J. (1985), Murine models of systemic lupus erythematosus. Adv. Immunol. 37: 269-390). Je 10 NZBW/F₁ Mäuse wurden wöchentlich entweder mit PBS (Kontrollgruppe) oder 100µg löslichem Fc-Rezeptor (sFcγRIIb) subkutan behandelt. Während von den Fc-Rezeptor behandelten Mäusen auch nach 40 Wochen noch alle Mäuse lebten, waren von der Kontrollgruppe nach diesem Zeitraum 70% verendet.

### Abb. 3: Kumulative Proteinurie von NZBW/F₁ Mäusen

Die Proteinurie bei erkrankten NZBW/F₁ Mäusen als Folge einer sich entwickelnden Glomerulonephritis ist ein wichtiges Kriterium für die Progression der Krankheit SLE. Von den wie in der Legende zu Abb. 1a beschrieben behandelten Mäusen wurde die Proteinurie (> 0,3 g/l Urin) bestimmt und kumulativ aufgetragen.

### Abb. 4: Krankheitsverlauf der Adjuvanz induzierten Arthritis (AIA) in Mäusen

Die AIA stellt ein anerkanntes Tiermodell für die Rheumatoide Arthritis dar. Die entzündliche Reaktion wird durch Gabe von Antigen in ein Gelenk ausgelöst. Die Behandlung erfolgt wöchentlich mit 100 µg löslichem FcγR IIb intraperitoneal (IP). (Nach: Waksman, B.H., Immune regulation in adjuvant disease and other arthritis models: relevance to pathogenesis of chronic arthritis, 2002, Scand. J. Immunol. 56(1): 12-34; Holmdahl, R., Lorentzen, J.C., Lu, S., Olofsson, P., Wester, L., Holmberg, J. & Pettersson, U., 2001, Arthritis induced in rats with nonimmunogenic adjuvants as models for rtheumatoid arthritis, Immunol. Rev. 184: 184-202).

### Abb. 5: IgG vermittelte Immunkomplex (IK)-induzierte Alveolitis

IK-Alveolitis ist ein anerkanntes Tiermodell für entzündliche Krankheiten, die mit hoher IK-Last einhergehen. Zur Induktion wird Mäusen ein Antigen intraperitoneal (IP) injiziert und ein gegen dieses Antigen gerichteter Antikörper intratracheal (IT) verabreicht. Als Folge davon bilden sich Immunkomplexe in der Lunge, die in entzündlichen Reaktionen resultieren. Ausgewertet wird die Infiltration von Neutrophilen (PMN) und die Hämorrhagie. Die Reaktion in diesem Tiermodell kann noch verstärkt werden, wenn vorgeformte Immunkomplexe aus Antigen und Antikörper IT appliziert werden. Eine gleichzeitige Gabe von 100 µg FcγR IIb IP unterdrückt die entzündlichen Reaktionen nahezu vollständig. (Nach: Tanoue, M., Yoshizawa, Y., Sato, T., Yano, H., Kimula, Y. & Miyamoto, K., 1993, The role of complement-derived chemotactic factors in lung injury induced by preformed immune complexes. Int. Arch. Allergy Immunol. 101(1): 47-51; Yoshizawa, Y., Tanoue, M., Yano, H., Sato, T., Ohtsuka, M., Hasegawa, S. & Kimula, Y. 1991, Sequential changes in lung injury induced by preformed immune complexes. Clin. Immunol. Immunopathol. 61 (3): 376-386).

| | |
|---|---|
| SEQ ID NO.1 | zeigt die bevorzugte minimale Aminosäuresequenz von FcγR IIb, welche an den Termini gegebenenfalls verlängert werden kann. |
| | |
| SEQ ID NO.2 | zeigt die bevorzugte minimale Aminosäuresequenz von FcγR III, welche ebenfalls an den Termini verlängert werden kann. |
| | |
| SEQ ID NO.3 und SEQ ID NO.4 | zeigen derartige an den Termini verlängerte Rezeptorsequenzen. |

Seq ID NO: 1
AVLKLEPQWI NVLQEDSVTL TCRGTHSPES DSIQWFHNGN LIPTHTQPSY RFKANNNDSG
EYTCQTGQTS LSDPVHLTVL SEWLVLQTPH LEFQEGETIV LRCHSWKDKP LVKVTFFQNG
KSKKFSRSDP NFSIPQANHS HSGDYHCTGN IGYTLYSSKP VTITV

Seq ID NO: 2
AVVFLEPQWY SVLEKDSVTL KCQGAYSPED NSTQWFHNES LISSQASSYF IDAATVNDSG
EYRCQTNLST LSDPVQLEVH IGWLLLQAPR WVFKEEDPIH LRCHSWKNTA LHKVTYLQNG
KDRKYFHHNS DFHIPKATLK DSGSYFCRGL VGSKNVSSET VNITI

SEQ ID NO: 3
MGTPAAPPKA VLKLEPQWIN VLQEDSVTLT CRGTHSPESD SIQWFHNGNL IPTHTQPSYR
FKANNNDSGE YTCQTGQTSL SDPVHLTVLS EWLVLQTPHL EFQEGETIVL RCHSWKDKPL
VKVTFFQNGK SKKFSRSDPN FSIPQANHSH SGDYHCTGNI GYTLYSSKPV TITVQAPSSS
PMGII

SEQ ID NO: 4
MRTEDLPKAV VFLEPQWYSV LEKDSVTLKC QGAYSPEDNS TQWFHNESLI SSQASSYFID
AATVNDSGEY RCQTNLSTLS DPVQLEVHIG WLLLQAPRWV FKEEDPIHLR CHSWKNTALH
KVTYLQNGKD RKYFHHNSDF HIPKATLKDS GSYFCRGLVG SKNVSSETVN ITITQG

## Patentansprüche

1. Pharmazeutische Zusammensetzung in Form einer wässrigen Lösung, **dadurch gekennzeichnet, dass** sie rekombinant hergestellten, löslichen FcγR IIb aus eukaryontischen oder insbesondere prokaryontischen Expressionssystemen in einer Menge von 40 bis 4000 mg, vorzugsweise 100 bis 1000 mg und einer Konzentration von bis zu 50 mg/ml enthält und gegebenenfalls weitere pharmazeutisch annehmbare Hilfs- oder/und Trägerstoffe umfasst.

2. Pharmazeutische Zusammensetzung nach Anspruch 1, **dadurch gekennzeichnet, dass** sie in Form einer Injektionslösung vorliegt.

3. Pharmazeutische Zusammensetzung nach Anspruch 1 oder 2, **dadurch gekennzeichnet, dass** sie FcγR IIb gemäß SEQ ID NO.1 oder eine am N-oder/und C-Terminus mit geeigneten Sequenzen des Wildtyp-Proteins verlängerte Form, bevorzugt gemäß SEQ ID NO. 3, enthält, wobei eine Sequenz des Wildtyp-Proteins für eine verlängerte Form geeignet ist, wenn die Sequenz keine Antigenizität hervorruft.

4. Verwendung einer pharmazeutischen Zusammensetzung gemäß einem der Ansprüche 1 bis 3 zur Herstellung eines Medikaments zur Behandlung von Krankheiten oder Zuständen, welche durch überschießende Immunreaktionen und krankhaft vermehrte Bildung von Antikörpern, insbesondere Autoantikörpern verursacht sind.

5. Verwendung einer pharmazeutischen Zusammensetzung zur Herstellung eines Medikaments nach Anspruch 4, **dadurch gekennzeichnet, dass** die Krankheit Multiple Sklerose, Systemischer Lupus erythematosus, Rheumatoide Arthritis oder eine Erkrankung ist, die mit einer erhöhten Anzahl von NK-Zellen einhergeht.

6. Verwendung einer pharmazeutischen Zusammensetzung zur Herstellung eines Medikaments nach Anspruch 4 oder 5, **dadurch gekennzeichnet, dass** das Medikament injiziert wird.

## Claims

1. Pharmaceutical composition in the form of an aqueous solution, **characterized in that** it contains recombinantly produced, soluble FcγR IIb from eukaryotic or in particular prokaryotic expression systems in an amount of 40 to 4000 mg, preferably 100 to 1000 mg and at a concentration of up to 50 mg/ml and optionally comprises other pharmaceutically acceptable adjuvants or/and excipients.

2. Pharmaceutical composition as claimed in claim 1, **characterized in that** it is present in the form of an injection solution.

3. Pharmaceutical composition as claimed in claim 1 or 2, **characterized in that** it contains FcγR IIb according to SEQ ID NO. 1 or a form that is extended at the N-terminus or/and C-terminus with suitable sequences of the wild-type protein, preferably according to SEQ ID NO.3, wherein a sequence of the wild-type protein is suitable for an extended form, if the sequence does not result in antigenicity.

4. Use of a pharmaceutical composition as claimed in one of the claims 1 to 3 for the manufacture of a medicament for the treatment of diseases or conditions which are caused by overshooting immune reactions and pathologically increased formation of antibodies and especially of autoantibodies.

5. Use of a pharmaceutical composition for the manufacture of a medicament according to claim 4, **characterized in that** the disease is multiple sclerosis, systemic lupus erythematosus, rheumatoid arthritis or a disease that is associated with an increased number of NK cells.

6. Use of a pharmaceutical composition for the manufacture of a medicament according to claim 4 or 5, **characterized in that** the medicament is to be injected.

## Revendications

1. Composition pharmaceutique sous la forme d'une solution aqueuse, **caractérisée en ce qu'**elle contient un récepteur FcyR IIb soluble et produit par recombinaison, provenant de systèmes d'expression eucaryotes ou en particulier procaryotes, dans une quantité allant de 40 à 4000 mg, de préférence de 100 à 1000 mg et une concentration allant jusqu'à 50 mg/ml et **en ce qu'**elle comprend, le cas échéant d'autres substances de support et/ou substances auxiliaires pharmaceutiquement acceptables.

2. Composition pharmaceutique selon la revendication 1, **caractérisée en ce qu'**elle se présente sous la forme d'une solution injectable.

3. Composition pharmaceutique selon la revendication 1 ou 2, **caractérisée en ce qu'**elle contient un récepteur FcyR IIb selon SEQ ID NO.1 ou une forme prolongée au niveau de la terminaison N et/ou C avec des séquences appropriées de la protéine de type sauvage, de préférence selon SEQ ID NO.3, une séquence de la protéine de type sauvage étant appropriée pour une forme prolongée lorsque la séquence ne provoque pas d'antigénicité.

4. Utilisation d'une composition pharmaceutique selon l'une des revendications 1 à 3 pour fabriquer un médicament afin de traiter des maladies ou des états qui sont provoqués par des réactions immunitaires excessives et une formation anormalement élevée d'anticorps, en particulier d'auto-anticorps.

5. Utilisation d'une composition pharmaceutique pour fabriquer un médicament selon la revendication 4, **caractérisée en ce que** la maladie est la sclérose en plaques, le lupus érythémateux systémique, l'arthrite rhumatoïde ou une affection liée à un nombre accru de cellules NK.

6. Utilisation d'une composition pharmaceutique pour fabriquer un médicament selon la revendication 4 ou 5, **caractérisée en ce que** le médicament est injecté.
